# EUROPÄISCHE PATENTSCHRIFT

(11) **EP 2 832 383 B1**
(45) Veröffentlichungstag und Bekanntmachung des Hinweises auf die Patenterteilung: **30.08.2023**
(21) Anmeldenummer: 14002553.7
(22) Anmeldetag: 23.07.2014
(51) Int. Cl.: A61M 60/113, A61M 60/531, A61M 60/515, A61M 60/38, A61M 60/237

(54) **Anordnung mit einer Blutpumpe und einer Pumpensteuerung**
Assembly having a blood pump and a pump control
Agencement doté d'une pompe à sang et commande de pompe

(30) Priorität: 29.07.2013 DE 102013012433
(43) Veröffentlichungstag der Anmeldung: 04.02.2015
(73) Patentinhaber: Xenios AG, 74076 Heilbronn (DE)
(72) Erfinder: Matheis, Georg, DE - 74076 Heilbronn (DE); Gorhan, Holger, DE - 71101 Schönaich (DE)
(74) Vertreter: Graf von Stosch, Andreas

(56) Entgegenhaltungen:
- WO-A1-87/02897
- WO-A1-2014/007785
- WO-A2-87/02894
- DE-A1- 3 133 177
- DE-A1-102005 039 446
- DE-A1-102010 004 600
- DE-T2- 60 031 781
- GB-A- 1 551 808
- US-A- 3 927 981
- US-A1- 2004 034 272
- US-A1- 2004 068 220
- US-A1- 2012 078 031
- US-A1- 2013 343 954
- US-B2- 7 963 905

## Beschreibung

Die Erfindung betrifft eine Anordnung mit einer Blutpumpe und einer Pumpensteuerung, die einen Rechner aufweist, der ein Steuersignal in ein Pumpenantriebssignal umwandelt.

Derartige Anordnungen werden beispielsweise für eine extrakorporale Kreislaufunterstützung (ECLS) eingesetzt.

Anwendungsgebiete der ECLS sind beispielsweise Patienten mit kardiogenem Schock oder einer Dekompensation einer Herzinsuffizienz, deren Herz nichtmehr dazu in der Lage ist, den Organismus ausreichend mit sauerstoffreichem Blut zu versorgen.

DE 600 31 781 T2 offenbart die Merkmale des Oberbegriffes des Anspruchs 1. Der Erfindung liegt die Aufgabe zugrunde, eine derartige Anordnung weiterzuentwickeln und ein Verfahren zum Betrieb einer Blutpumpe vorzustellen.

Diese Aufgabe wird mit einer gattungsgemäßen Anordnung gelöst, bei der das Pumpenantriebssignal eine wellenartig an- und abschwellende Pumpenleistung für eine pulsatile Strömung bewirkt. Die durch das Pumpenantriebssignal erzeugte pulsatile Strömung verbessert die Kreislaufsituation.

Unter wellenartig an- und abschwellender Pumpenleistung ist kein konstanter Pumpenhub und auch kein An- und Abschalten der Pumpe zu verstehen, sondern eine über die Zeit variierte Pumpenleistung, die durch ein variables Steuersignal bewirkt wird.

Die Anordnung ermöglicht ein Herzunterstützungssystem, das in den Herzzyklus integrierte Pulse abgibt, um die Durchblutung der Herzkranzgefäße zu verbessern und das Herz besser mit Sauerstoff zu versorgen.

Vorteilhaft ist es wenn die Blutpumpe zusätzlich eine konstante Grundleistung bereitstellt. Dadurch wird der systemische Perfusionsdruck mit einen laminaren Basisfluss erhöht.

Diese konstante Grundleistung kann durch die Pumpe bereitgestellt werden, die auch für die pulsatile Strömung sorgt. Je nach Anwendungsgebiet kann es vorteilhaft sein, dass die Anordnung eine weitere Blutpumpe aufweist, die die konstante Grundleistung bereitstellt.

In diesem Fall kann die weitere Pumpe auch ein wellenartig an- und abschwellende Pumpenleistung bereitstellen.

Somit kann entweder mit einer Pumpe die pulsatile Strömung und die konstante Grundleistung bereitgestellt werden oder die Funktionen der an- und abschwellenden Pumpenleistung und der konstanten Grundleistung werden auf zwei Pumpen verteilt.

Es können jedoch auch zwei Pumpen verwendet werden, die jeweils eine wellenartig an- und abschwellende Pumpenleistung bereitstellen. Dies ermöglicht es, mit einer zweiten Blutpumpe zeitverzögert zu einer ersten Blutpumpe eine wellenartig an- und abschwellende Pumpenleistung bereitzustellen, sodass sich die Druckwellen überlagern.

Die beanspruchte Anordnung weist einen Oxygenator auf, der von der Pumpe versorgt wird. Die Pumpe ist dabei vor dem Oxygenator angeordnet. Vorteilhaft ist es, wenn eine Blutpumpe in Strömungsrichtung vor dem Oxygenator und eine weitere Blutpumpe nach dem Oxygenator angeordnet ist.

Eine bevorzugte Ausführungsvariante sieht vor, dass der Oxygenator ein Gehäuse aufweist und mindestens eine Blutpumpe in diesem Gehäuse angeordnet ist. Dies ermöglicht es, beispielsweise in dem Gehäuse des Oxygenators vor dem Oxygenator und hinter dem Oxygenator eine Blutpumpe anzuordnen.

Eine besonders vorteilhafte Ausführungsvariante sieht vor, dass die Anordnung mindestens eine nicht okklusive Blutpumpe, wie insbesondere eine Diagonal-, Axial- oder Zentrifugalpumpe aufweist.

Um das benötigte Steuersignal bereitzustellen weist die Anordnung einen Taktgeber auf. Dieser Taktgeber kann nach einem vorbestimmten Rhythmus das Steuersignal für die Pumpe im Hinblick auf Frequenz und Amplitude bereitstellen. Dadurch wird die wellenartig an- und abschwellende Pumpenleistung erreicht.

Dieses Steuersignal wird bei der beanspruchten Ausführungsvariante durch ein EKG bereitgestellt. Dazu wird eine Software mit der Möglichkeit zur Erfassung eines EKG-Signals in die Steuereinheit eines ECLS-Systems integriert. Ein Patientenkabel leitet das EKG-Signal am Patienten ab. Die so erfasste R-Zacke der Taktgeber (Trigger) ist für die Abgabe eines Softwaretriggers zum Start der Blutpumpe, welche dann den Puls generiert. Die Software sorgt für die exakte Abgabe des Pulses in die Diastole. Dabei wird vorzugsweise darauf geachtet, dass die Dauer des Pulses so angepasst ist, dass der Puls bei Beginn der Systole nicht mehr vorhanden ist. Es kann aber auch ein Pulsprofil erzeugt werden, das sich auf die Systole und/oder auf die Diastole auswirkt.

Kumulativ oder alternativ wird vorgeschlagen, dass die Anordnung einen arteriellen Drucksensor aufweist, der das Steuersignal bereitstellt. Dies ermöglicht es, mittels einer Druckmessung an einer Arterie auf die Pumpenleistung einzuwirken.

Die Erfahrungen haben gezeigt, dass es vorteilhaft ist, wenn die Anordnung eine arterielle Kanüle aufweist, die länger als etwa 20 cm, vorzugsweise länger als 30 cm ist. Die besonders lange Kanüle dient dazu, den Puls so nah wie physiologisch möglich am Herzen abgeben zu können.

Die der Erfindung zugrunde liegende Aufgabe wird auch mit einem Verfahren zum Betreib einer Blutpumpe gelöst, bei dem die Pumpe mit einer iterierenden Leistung betrieben wird, um eine wellenartig an- und abschwellende pulsatile Strömung zu erzielen.

Dabei kann Phasenverschoben zur pulsatilen Strömung eine weitere Blutpumpe eine wellenartig an- und abschwellende Pumpenleistung bewirken.

Vorteilhaft ist es, wenn der pulsatilen Strömung mindestens einer Pumpe eine Grundlast überlagert wird.

Bei der Verfahrensführung wird vorzugsweise darauf geachtet, dass mit der Pumpe der diastolische Druck erhöht wird. Dies ermöglicht es, mit einem ECLS-System die Kreislaufunterstützung so zu generieren, dass zusätzlich zu einem laminaren Basisfluss die pulsatile Funktion so eingestellt wird, dass in der Phase der Diastole eine Fluss- und Druckerhöhung stattfindet. Die Triggerung des Systems erfolgt dabei vorzugsweise durch eine Synchronisation mit dem Herzen.

Die beschriebene Anordnung kann aber auch dazu verwendet werden, mit der Pumpe einen Oxygenator anzuströmen. Die Pulsatilität verbessert dabei Funktion und Lebensdauer des Oxygenators.

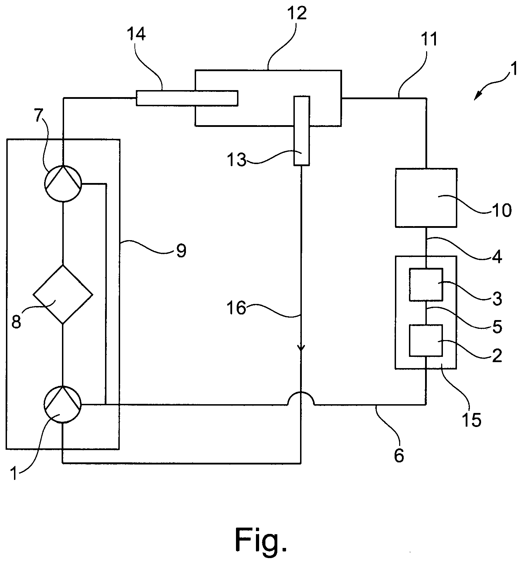

Ein Ausführungsbeispiel einer derartigen Anordnung ist in der Zeichnung dargestellt und wird im Folgenden näher erläutert. Es zeigt die einzige Figur schematisch die einzelnen Elemente der Anordnung und ihre Verschaltung.

Wesentliche Elemente der Anordnung 1 sind eine erste Blutpumpe 1, eine Pumpensteuerung 2 und ein Rechner 3. Der Rechner 3 wandelt ein Steuersignal 4 in ein Pumpenantriebssignal um. Dieses Pumpenantriebssignal 5 bewirkt über die Pumpensteuerung 2 eine wellenartig an- und abschwellende Pumpenleistung an der Pumpe 1, die dadurch eine pulsatile Strömung bewirkt.

Die Pumpensteuerung 2 steht über die Leitung 6 mit der ersten Pumpe 1 und einer weiteren Pumpe 7 in Verbindung. Dies ermöglicht es mit der ersten Pumpe 1, die vor einem Oxygenator 8 angeordnet ist, sowohl Grundlast als auch pulsatile Strömung zu erzeugen. Es kann jedoch auch mit der ersten Pumpe 1 vor dem Oxygenator 8 eine Grundlast erzeugt werden und mit der zweiten Pumpe 7 nach dem Oxygenator 8 eine pulsatile Strömung.

Letztlich kann auch mit der ersten Pumpe 1 vor dem Oxygenator 8 und der zweiten Pumpe 7 nach dem Oxygenator jeweils eine pulsatile Strömung erzielt werden. Dies ermöglicht es, zeitverzögerte Wellen aufgrund der Distanz der Pumpen zueinander zu überlagern oder die Pumpen mit zeitverzögerten Signalen anzusteuern.

Die Pumpen 1 und 7 sind zusammen mit dem Oxygenator 8 in einem Gehäuse 9 angeordnet. Dies ermöglicht einen einfachen Aufbau. Im dargestellten Ausführungsbeispiel führt auch nur eine Leitung 6 von der Pumpensteuerung 2 zum Gehäuse 9, um im Gehäuse 9 die zwei Pumpen 1 und 7 mit einem Pumpenantriebssignal zu versorgen. Als Alternative kann auch jeweils eine Leitung zur ersten Pumpe 1 und eine weitere Leitung zur zweiten Pumpe 7 geführt werden.

Als Blutpumpe wird zumindest für die erste Pumpe 1 eine Diagonalpumpe verwendet. Vorzugsweise sind beide Pumpen 1 und 7 als Diagonalpumpe ausgebildet. Es können jedoch auch Axial- oder Zentrifugalpumpen eingesetzt werden.

Das Steuersignal 4 wird von einem EKG 10 bereitgestellt, das über ein Kabel 11 mit einem Patienten 12 in Verbindung steht.

Im Blutkreislauf oder im Herz des Patienten 12 liegen eine venöse Kanüle 13 und eine arterielle Kanüle 14. Die arterielle Kanüle ist dabei etwa 35 - 40 cm, vorzugsweise 30 bis 45 cm, lang und die venöse Kanüle wird in die große Hohlvene gelegt.

Beim Betrieb des ECLS-Systems wird mit dem EKG 10 über die Leitung 11 ein EKG-Signal eines Patienten 12 erfasst, um ein Steuersignal 4 zu erzeugen. Dieses Steuersignal 4 wird über den Rechner 3 in ein Pumpensignal 5 umgewandelt, das über die Pumpensteuerung 2 und die Leitung 6 die Pumpen 1 und 7 steuert oder mit einem Strom versorgt. Dabei wird eine Konsole 15 eingesetzt die einen SW-Trigger zum Start der Blutpumpe 1 nach einem speziell entwickelten Algorithmus mit dem Ziel abgibt, Impulse in die Systole und/oder die Diastole abzugeben.

Dazu wird das EKG-Signal in die Konsole implementiert. Das User Interface wird angepasst, um Einstellmöglichkeiten für das EKG zu schaffen und um einen Markerkanal zur Darstellung der jeweiligen Aktion der Blutpumpe als Sense oder Puls darzustellen.

Im Blutkreislauf 16 von der venösen Kanüle 13 zur arteriellen Kanüle 14 wird im Oxygenator 8 das Blut mit Sauerstoff angereichert und CO₂ entfernt.

Die Blutpumpe 1 wird um einen speziellen Wert on top zur Basisdrehzahl für einen definierten Zeitraum innerhalb eines maximalen Zeitfensters beschleunigt, welches in Abhängigkeit zur aktuellen Herzfrequenz steht. Die zeitliche Begrenzung erfolgt durch einen weiteren Algorithmus.

Die Blutpumpe oder die Blutpumpen 1 und 7 werden so gesteuert, dass eine diastolische Augmentation eintritt. Während dieser Herzaktion wird der koronare Perfusionsdruck erhöht. Anschließend fällt der enddiastolische Blutdruck im herznahen Bereich der Aorta auf einen tieferen Wert als normal. Die folgende Systole hat weniger Auswurfwiderstand zu überwinden und heißt deshalb "beeinflusste Systole". Die niedrigere Nachlast erkennt man am geringeren systolischen Druck.

Durch das Anheben des diastolischen Drucks wird die Sauerstoffbilanz des Herzmuskels auf zwei Wegen verbessert: die myokardiale Sauerstoffversorgung wird durch eine Steigerung des koronaren Perfusionsdrucks erhöht und gleichzeitig wird die mechanische Herzarbeit und somit der myokardiale Sauerstoffverbrauch vermindert. Dadurch werden die Voraussetzungen für eine Erholung des Herzens gebessert.

Ein Problem von Oxygenatoren ist das Clotting. Dabei lagern sich Blutbestandteile auf der Gasaustauschmembran ab. Außerdem können in wenig durchströmten Arealen des Oxygenators, Koagel entstehen. Durch die pulsatile Durchströmung des Oxygenators wird die Anströmung im Oxygenator verändert und dadurch wird die Haltbarkeit des Oxigenators verbessert.

Außerdem wird als Nebeneffekt die Gasaustauschleistung verbessert, da die Grenzschicht zwischen Faser und strömendem Blut verringert wird.

## Patentansprüche

1. Anordnung für eine extrakorporale Kreislaufunterstützung (ECLS) eines Patienten (12) mit einer Blutpumpe (1) und einer Pumpensteuerung (2), wobei die Anordnung einen Oxygenator (8) aufweist, der von der Blutpumpe (1) versorgt wird, wobei die Blutpumpe (1) in Strömungsrichtung vor dem Oxygenator angeordnet ist, und wobei die Anordnung einen Rechner (3) aufweist, der ein Steuersignal (4) in ein Pumpenantriebssignal (5) umwandelt, wobei das Pumpenantriebssignal (5) eine wellenartig an- und abschwellende Pumpenleistung für eine pulsatile Strömung bewirkt, wobei die Anordnung ein EKG (10) mit einem Taktgeber aufweist, der das Steuersignal (4) bereitstellt und wobei eine Software zur Erfassung eines EKG-Signals in die Steuereinheit (3) der Anordnung integriert ist, die über ein Patientenkabel (11) das EKG-Signal des Patienten (12) erfasst, um das Steuersignal (4) zu erzeugen,
***dadurch gekennzeichnet, dass*** der Taktgeber eine R-Zacke des EKGs (10) des Patienten (12) ist, der die Abgabe eines Softwaretriggers zum Start der Blutpumpe veranlasst, die dann einen Strömungspuls generiert, so dass die Software die Abgabe des Strömungspulses in die Diastole des Herzzyklus des Patienten (12) veranlasst.

2. Anordnung nach Anspruch 1, ***dadurch gekennzeichnet, dass*** die Dauer des Strömungspulses so angepasst wird, dass der Puls bei Beginn der Systole nicht mehr vorhanden ist.

3. Anordnung nach Anspruch 1 oder 2, ***dadurch gekennzeichnet, dass*** die Blutpumpe (1) zur Erzeugung des Strömungspulses für einen definierten Zeitraum innerhalb eines maximalen Zeitfensters über eine Basisdrehzahl hinaus beschleunigt wird, wobei das maximale Zeitfenster in Abhängigkeit zur Herzfrequenz des Patienten (12) steht.

4. Anordnung nach einem der vorhergehenden Ansprüche, ***dadurch gekennzeichnet, dass*** die Blutpumpe (1) derart gesteuert wird, dass beim Patienten (12) eine diastolische Augmentation eintritt.

5. Anordnung nach einem der vorhergehenden Ansprüche, ***dadurch gekennzeichnet, dass*** die Blutpumpe (1) zusätzlich eine konstante Grundleistung bereitstellt.

6. Anordnung nach einem der vorhergehenden Ansprüche, ***dadurch gekennzeichnet, dass*** der Oxygenator (8) ein Gehäuse (9) aufweist und mindestens eine Blutpumpe (1, 7) in diesem Gehäuse (9) angeordnet ist.

7. Anordnung nach einem der vorhergehenden Ansprüche, ***dadurch gekennzeichnet, dass*** sie mindestens eine nichtokklusive Blutpumpe (1) wie insbesondere eine Diagonal-, Axial- oder Zentrifugalpumpe aufweist.

8. Anordnung nach einem der vorhergehenden Ansprüche, ***dadurch gekennzeichnet, dass*** sie einen arteriellen Drucksensor aufweist, der das Steuersignal (4) bereitstellt.

9. Anordnung nach einem der vorhergehenden Ansprüche, ***dadurch gekennzeichnet, dass*** sie eine arterielle Kanüle (14) aufweist, die länger als etwa 20 cm ist.

10. Anordnung nach Anspruch 9, ***dadurch gekennzeichnet, dass*** die arterielle Kanüle (14) eine Länge von 30 bis 45 cm, vorzugsweise von 35 bis 40 cm, aufweist.

## Claims

1. Arrangement for an extracorporeal circuit support (ECLS) of a patient (12) with a blood pump (1) and pump control unit (2), wherein the arrangement has an oxygenator (8) which is supplied by the blood pump (1), wherein the blood pump (1) is arranged in the direction of flow upstream of the oxygenator, and wherein the arrangement has a computer (3) that converts a control signal (4) into a pump actuating signal (5), wherein the pump actuating signal (5) produces a wave-like surging and subsiding pump output for a pulsatile flow, wherein the arrangement has an ECG (10) with a clock generator which provides the control signal (4), and wherein software with the ability to record an ECG signal is integrated into the control unit (3) of the arrangement, which records the ECG signal of the patient (12) by means of a patient cable (11) for generating the control signal (4), **characterized in that** the clock generator is the recorded R wave of an ECG (10) of the patient (12) causing delivery of a software trigger for starting the blood pump which then generates the pulse, such that the software ensures the delivery of the pulse during the diastole of the cardiac cycle of the patient (12).

2. Arrangement according to claim 1, **characterized in that** the duration of the pulse is adapted in such a way that at the start of systole the pulse is no longer present.

3. Arrangement according to claims 1 or 2, **characterized in that** the blood pump (1) for generating the pulse is accelerated beyond the base speed for a defined period within a maximum time window which is dependent on the heart frequency of the patient (12).

4. Arrangement according to claim 1, **characterized in that** the blood pump (1) is controlled in such a way that a diastolic augmentation at the patient (12) occurs.

5. Arrangement according to any one of the preceding claims, **characterised in that** the blood pump (1) also provides a constant basic output.

6. Arrangement according to any one of the preceding claims, **characterised in that** the oxygenator (8) has a housing (9) and **in that** at least one blood pump (1, 7) is arranged in this housing (9).

7. Arrangement according to any one of the preceding claims, **characterised in that** is has at least one non-occlusive blood pump (1) such as, in particular, a diagonal, axial or centrifugal pump.

8. Arrangement according to any one of the preceding claims, **characterised in that** it has an arterial pressure sensor which provides the control signal (4).

9. Arrangement according to any one of the preceding claims, **characterised in that** it has an arterial cannula (14) which is longer than 20 cm.

10. Arrangement according to claim 9, **characterised in that** the arterial cannula (14) has a length of from 30 cm to 45 cm, preferably from 35 to 45 cm.

## Revendications

1. Agencement d'assistance à la circulation extracorporelle (ECLS) d'un patient (12) comprenant une pompe à sang (1) et une commande de pompe (2), dans lequel l'agencement comprend un oxygénateur (8), qui est alimenté par la pompe à sang (1), dans lequel la pompe à sang (1) est disposée avant l'oxygénateur dans le sens de l'écoulement, et dans lequel l'agencement comprend un calculateur (3), qui convertit un signal de commande (4) en un signal d'entraînement de pompe (5),
dans lequel le signal d'entraînement de pompe (5) produit une puissance de pompage augmentant et diminuant de façon ondulée pour un écoulement pulsatile,
dans lequel l'agencement comprend un ECG (10) comprenant une horloge, qui délivre le signal de commande (4) et
dans lequel un logiciel pour recueillir un signal ECG est intégré dans l'unité de commande (3) de l'agencement, qui recueille, via un câble de patient (11), le signal ECG du patient (12), afin de générer le signal de commande (4),
**caractérisé en ce que** l'horloge est une onde R de l'ECG (10) du patient (12), qui provoque l'émission d'un déclencheur de logiciel pour démarrer la pompe à sang, qui génère ensuite une impulsion d'écoulement, de manière à ce que le logiciel provoque l'émission de l'impulsion d'écoulement dans la diastole du cycle cardiaque du patient (12).

2. Agencement selon la revendication 1, **caractérisé en ce que** la durée de l'impulsion d'écoulement est ajustée de telle manière que l'impulsion n'est plus présente au début de la systole.

3. Agencement selon la revendication 1 ou 2, **caractérisé en ce que** la pompe à sang (1) est accélérée au-delà d'une vitesse de rotation de base pour produire l'impulsion d'écoulement pendant un intervalle de temps défini dans un laps de temps maximal, dans lequel le laps de temps maximal est fonction de la fréquence cardiaque du patient (12).

4. Agencement selon l'une des revendications précédentes, **caractérisé en ce que** la pompe à sang (1) est commandée de manière à ce qu'une augmentation diastolique se produise chez le patient (12).

5. Agencement selon l'une des revendications précédentes, **caractérisé en ce que** la pompe à sang (1) fournit en outre une puissance de base constante.

6. Agencement selon l'une des revendications précédentes, **caractérisé en ce que** l'oxygénateur (8) comprend un boîtier (9) et au moins une pompe à sang (1, 7) est disposée dans ce boîtier (9).

7. Agencement selon l'une des revendications précédentes, **caractérisé en ce qu'**il comprend au moins une pompe à sang (1) non occlusive, telle qu'en particulier une pompe diagonale, axiale ou centrifuge.

8. Agencement selon l'une des revendications précédentes, **caractérisé en ce qu'**il comprend un capteur de tension artérielle, qui fournit le signal de commande (4).

9. Agencement selon l'une des revendications précédentes, **caractérisé en ce qu'**il comprend une canule artérielle (14) qui a une longueur supérieure à environ 20 cm.

10. Agencement selon la revendication 9, **caractérisé en ce que** la canule artérielle (14) a une longueur de 30 à 45 cm, de préférence de 35 à 40 cm.
